# EUROPEAN PATENT SPECIFICATION

(11) **EP 1 118 332 B1**
(45) Date of publication and mention of the grant of the patent: **09.03.2005**
(21) Application number: 00830035.2
(22) Date of filing: 20.01.2000
(51) Int. Cl.: A61K 45/06, A61P 19/02

(54) **A composition for treating degenerative joint diseases**
Zusammensetzung zur Behandlung von degenerativen Gelenkerkrankungen
Compositions pour le traitement d'affections dégénératives des articulations

(43) Date of publication of application: 25.07.2001
(73) Proprietor: Innovet Italia S.r.l., 20144 Milano (IT)
(72) Inventor: Della Valle, Francesco, 35122 Padova (IT); Della Valle, Federica, 35141 Padova (IT)
(74) Representative: Maggioni, Claudio

(56) References cited:
- WO-A-95/01096
- WO-A-98/33494
- DE-A- 3 607 302

## Description

The present invention relates to veterinary and pharmaceutical compositions which can be used for the prevention and/or the coadjuvant treatment of degenerative joint diseases in man and in animals and also diseases connected with pain in the periarticular restraining structures (muscles, tendons, ligaments).

Many paraphysiological and pathological conditions in dogs, and above all ageing, predispose towards degenerative joint diseases which are known as DJDs (Mortellaro et al., Veterinaria, 1 (suppl.): 1-40, 1999).

From a paraphysiological point of view, some breeds have a predisposition to develop DJD, in the sense that dogs of large and very large breeds are much more susceptible to joint disease because of the different maturation of the osteocartilaginous tissue in comparison with the musculo-ligamentous tissue, the latter being much slower (Grandjean et al., Rec. Med. Vet., 172: 519-530, 1996). As well as the predisposition of breeds there is a certain predisposition connected with lifestyle. Dogs which are exuberant and/or are used for sporting activities may undergo hyper-stressing of the joint area with mechanical and/or oxidative damage of the restraining structures of the joint including stretching of muscles and ligaments.

Finally, there is a long list of pathological conditions which include all those developmental clinical conditions which predispose to DJD, to which the acronym DOD (developmental orthopaedic disease) has recently been attributed (Richardson et al., Vet. Clin, of North Am.: Small Animal Practice 28: 115-135, 1998), and which include many developmental arthropathies affecting the shoulder (OCD), the elbow (for example, OCD, UAP, FCP), the carpus, (RCC, hyperextension), the knee (OCD, dislocation of the kneecap, *genu valgum*), the ankle or hock (OCD, *pes varu,* lateral twisting), the foot (for example, sesamoid diseases), and the hip (dysplasia, Legg Calvè Perthes disease) (Thomson et al., Australian Vet. J., 72(10), 1995; Mitsuoka et al., Arthroscopy, 14(6): 630-633, 1998; Alexander, Vet. Clin, of North Am.: Small Animal Practice, 22(3): 503-511, 1992; Cardinet et al., JAVMA, 210(10): 1466-1473, 1997; Fluckiger et al., Zentralbl. Veterinarmed. A., 45(5): 199-207, 1998; Langenbach et al., J. Am. Vet. Med. Assoc., 213 (10) : 1439-1443, 1998).

It should be noted that the pathological conditions listed above are not specific to dogs alone but also affect horses and other animals, as well as man.

It has been found that the preventive or therapeutic treatment of arthritic symptoms cannot be limited purely to chondroprotective intervention but should also aim to optimize and/or to re-establish the restraining functionality of the periarticular soft tissues and, in the first place, muscles and ligaments, which together can be defined as myo-tendinous-ligamentous morphofunctional units (MTL-MFUs).

The periarticular restraining structures (muscles, tendons, ligaments) in fact have a primary role in rendering the load on the joint homogeneous and consequently preventing the triggering and/or progress of degenerative phenomena. Frequently, however, these structures may not be sufficiently effective in correctly reducing or damping the load bearing on the cartilage. This may be due to many causes such as, for example:
- "wear and tear" damage of the soft tissues due to excessive competitive/sports activity or to ageing;
- delay in myo-ligamentous maturation which is typical of puppies of large breeds;
- obesity to which puppies of medium small breed are prone because of early maturation of the adipose tissue;
- microtrauma connected with physical activity (traumatic stress on the cartilage);
- loosening of the ligaments which frequently accompanies DODs;
- muscular atrophy resulting from dysplasic pain;
- rupture of the cruciate ligament which is typical of large breeds.

This results in a need to provide treatments which can protect and/or re-equilibrate the joint region as a whole, rather than being limited to a single component.

Chondroprotection, which has been known for some years as an intervention which can protect the joint cartilage from degenerative damage connected with arthrosis, represents an equilibrating approach since it can stimulate the chondrocyte biosynthetic (anabolic) pathways, at the same time inhibiting the degradative (catabolic) pathways which are responsible for cartilage degeneration (Burkhardt et al., Sem. Arth. Rheum., 17 (suppl. 1): 3-34, 1987). Clinically, a chondroprotector is defined as a compound which can slow the progress of the main radiological signs of osteoarthritis and can prevent the appearance of new arthritic symptoms.

Amongst the substances with chondroprotective activity, particular attention has been given, for some time, to chondroitin sulphate (CS). As a mucopolysaccharide which is very concentrated at cartilaginous level, where it constitutes the main glycosaminoglycan (GAG) present in the matrix proteoglycans (PGs), CS incurs troublesome qualitative-quantitative alterations in the course of arthritic degeneration. Its use as a chondroprotector is justified not only by the fact that it represents the main starting product for the synthesis of proteoglycans, but also by its pro-anabolic activities, since it can stimulate the synthesis of GAGs and of collagen and can protect the matrix macromolecules from degradation (enzymatic and non-enzymatic) which they incur during arthrosis (Bassler et al., Int. J. of Tissue Reac., 14: 231-241, 1992).

CS combines, with its biological action of reequilibration between the activities of synthesis and metabolic degradation of the chondrocytes, physico-chemical and pharmacokinetic characteristics such as to suggest its advantageous therapeutic application.

The use of specific fractions with low molecular weights (average molecular weight of 5,000-10,000 Daltons) but with a degree of sulphation which can be physiologically superimposed on that of the endogenous CS in fact not only achieves a high absorption and a selective concentration at joint level, but also achieves maximum stimulation of the tissue repair capabilities. It has also been found that the elimination of CS molecules having molecular weights less than or equal to 3,000 Daltons leads to a considerable advantage which is that of preventing an undesired collagenolytic effect associated with these molecules.

DL-alpha-lipoic acid (ALA), which is also known as thioctic acid and is defined chemically as 1,2-dithiolano-3-pentanoic acid, is a disulphide derivative of octanoic acid which has been known for decades to be a crucial prosthetic group of various cell enzymatic systems which are involved, in particular, in the pyruvate oxidative "pathway", in the Krebs cycle, and in the metabolism of amino-acids (degradation and biosynthesis) (Bustamente et al., Free Radical Biol and Ned., 24: 1023-1039, 1998).

ALA, as such or in its reduced form (DHLA, dihydrolipoic acid), is a powerful antioxidant, since it performs a "radical scavenger" activity in relation to numerous free radicals (ROS, reactive oxygen species), such as the oxygen singlet, the hydroxyl radical, hypochlorous acid (HOCl), nitrogen monoxide, and peroxynitrite. This antioxidant effect is also amplified by the physico-chemical characteristics of the compound which make it a substance which is soluble both in the aqueous phase and in the lipid phase.

After reduction to DHLA, ALA is also capable of contributing to the non-enzymatic regeneration of endogenous antioxidants of crucial importance, such as reduced glutathione (GSH). It also stimulates the intracellular synthesis of GSH, thus increasing the endogenous reserves thereof.

The problem upon which the present invention is based is that of providing a novel treatment for degenerative joint diseases, both in animals and in man.

The approach proposed provides for intervention, not only on the joints, but on the whole periarticular area, that is, on the myo-tendinous-ligamentous morphofunctional units (MTL-MFUs).

This problem is solved by a composition as described in the appended claims.

The composition of the present invention comprises a mixture of chondroitin sulphates and of an active ingredient selected from DL-alpha-lipoic acid, a pharmaceutically acceptable salt, ester or amide thereof, or an optical isomer thereof, in a proportion by weight of chondroitin sulphate/active ingredient variable from 50:1 to 1:2, preferably from 10:1 to 1:1. This composition has been found particularly useful in the treatment of pathological conditions affecting the MTL-MFU, by virtue of the synergistic action of chondroitin sulphate, which is particularly active on the joints, and of DL-alpha-lipoic acid (or its derivatives), which is active both on the joints and on the periarticular tissues.

The chondroitin sulphates used are normally a mixture of chondroitin 4- and 6-sulphates having a degree of sulphation per disaccharide of between 0.60 and 1.

For the purposes of the present invention, the chondroitin sulphate will be a particular molecular fraction having an average molecular weight of between 5,000 and 10,000 Daltons which is free of chondroitin sulphates with molecular weights less than or equal to 3,000 Daltons. These molecular fractions of chondroitin sulphates have a degree of sulphation per disaccharide of between 0.75 and 1 and a protein content of less than 1.5%, preferably between 0.05% and 1.2%. The above-mentioned molecular fractions of chondroitin sulphates can be produced by purification of commercial chondroitin sulphate, as will be described below.

DL-alpha-lipoic acid may be used as such or in salified form, for example, as a salt with an alkali-metal or an alkaline-earth metal, preferably as a sodium salt. Alternatively, a pharmaceutically acceptable ester or amide of ALA, preferably DL-alpha-lipoamide, may be used. The D optical isomers (the naturally existing form) or the L optical isomers of thioctic acid may also be used for the purposes of the present invention.

Thioctic acid and its derivatives are commercial products or can be prepared from commercial products by methods well known to experts in the art.

The composition of the present invention may also comprise pharmaceutically acceptable excipients selected according to the type of formulation and to the desired method of administration, as will be described further below.

In addition, the composition of the present invention may further comprise one or more active substances selected from:
- an amino sugar, preferably glucosamine,
- a manganese salt, preferably manganese ascorbate,
- an antioxidant selected from vitamin E, vitamin C and flavonoids.

The amino sugar, if present, will preferably be in a ratio by weight, relative to the chondroitin sulphate, of between 1:1 and 4:1.

The manganese salt, if present, will preferably be in a ratio by weight, relative to the chondroitin sulphate, of between 1:3 and 1:10.

The vitamin E, if present, will preferably be in a ratio by weight, relative to the chondroitin sulphate, of between 1:7 and 1:3.

The vitamin C, if present, will preferably be in a ratio by weight, relative to the chondroitin sulphate, of between 1:10 and 1:1.

The flavonoid, which is preferably quercetin or rutin, if present, will preferably be in a ratio by weight, relative to the chondroitin sulphate, of between 1:5 and 1:1.

The amino sugar, together with the chondroitin sulphate, is a substrate for the synthesis of collagens and proteoglycans.

Manganese is a cofactor of the glycosyltransferases and thus plays an important part in the synthesis of glycosaminoglycans.

The antioxidants coadjuvate the thioctic acid in its effect in repairing oxidative damage at joint level.

Thioctic acid, as stated, can act both at joint level and at the level of the skeletal musculature, thus characterizing the proposed novel therapeutic approach to the MTL-MFU.

### Biological and clinical examples

### A - EVALUATION OF THE CHONDROPROTECTIVE ACTIVITY OF VARIOUS MOLECULAR FRACTIONS OF CHONDROITIN SULPHATE "IN VITRO"

### Method and materials

Cultures of differentiated dog joint chondrocytes were used in accordance with the method described, for human chondrocytes, by Bassler (Bassler et al., In Vitro, 22, 113, 1986; Bassler et al., Biochem. Pharmacol., 37, 1939, 1988).

The joint cartilage was withdrawn by arthroscopy from the knee joints of male German Shepherd dogs aged 7 years, weighing 35 kg, which had lameness in the right hind leg and for which a previous episode of transitory lameness had been reported at the age of six years.

The cartilage was immediately digested with 1 mg/ml of collagenase from histolytic Clostridium (Fluka Cod. 27680) in pH 7.4 sodium carbonate/bicarbonate buffer for 24 hours.

The cells were seeded at a rate of 10⁶ per 2 ml of culture medium and were kept rotating (100 revolutions/minute) at 37°C in at atmosphere of 95% air and 5% CO₂.

The cells were grown for 8 days. The addition of the molecular fractions of chondroitin sulphate to be tested - which were dissolved in pH 7.4 sodium carbonate/bicarbonate buffer at a concentration of 10 mg/ml - took place on the 4th day so that it was possible to reckon on a period of 4 days of incubation of the fractions to be tested with the cells.

The following compounds were tested:
a) a molecular fraction of chondroitin sulphate with an average molecular weight of less than 5,000 Daltons (compound D);
b) a molecular fraction of chondroitin sulphate with an average molecular weight of between 5,000 and 10,000 Daltons (compound B);
c) a molecular fraction of chondroitin sulphate with a molecular weight of between 10,000 and 20,000 Daltons (compound C);
d) a molecular fraction of chondroitin sulphate with a molecular weight greater than 20,000 Daltons (compound E).

Collagenolytic activity was measured, as a parameter indicative of the chondroprotective activity, with the use of a NEN-KIT (NEK-016 from NEN Corp.) and by quantifying it as production of soluble radioactive fragments, separated rapidly by centrifuging from undigested collagen fibrils.

The results are shown in Table I

**Table I**

| Compound tested | Number of tests | collagenolytic activity (mg/mg DNA) |
|---|---|---|
| buffer alone | 5 | 35 ± 4 |
| compound D | 5 | 40 ± 6 |
| compound B | 5 | 15 ± 2 |
| compound C | 5 | 18 ± 3 |
| compound E | 5 | 24 ± 5 |

As can be seen from the data given, whereas the molecular fraction of chondroitin sulphate with an average molecular weight of less than 5,000 Daltons had an extremely high collagenolytic activity, the higher molecular fractions showed this activity to a lesser extent. In particular, the molecular fraction between 5,000 and 10,000 Daltons, from which the CS molecules with molecular weights less than or equal to 3,000 Daltons had been eliminated by the method described below, was characterized by lower collagenolytic activity and thus constitutes the preferred fraction.

### B - CLINICAL EVALUATION OF THE COMBINATION OF MOLECULAR FRACTIONS OF CHONDROITIN SULPHATE AND DL-a-LIPOIC ACID ON JOINT FUNCTIONALITY AS A WHOLE IN DOGS

### Method and materials

In order to evaluate the joint functionality as a whole and the effect of various treatments thereon *in vivo*, a combined method of clinical investigation, investigation with instruments and biochemical investigation was used, and was applied to large, generally adult dogs, having degenerative joint disease (DJD) secondary to rupture of the cruciate ligament, to dislocation of the kneecap, or to genu valgum.

The method used comprised the following investigations:
(i) clinical investigation of pain upon palpation as described by Bouck (G.R. Bouck et al., Veterinary and Comparative Orthopaedic Traumatology, 8, 177-83, 1995). The scale of values used was from 1 to 4 and, in particular:
   1 = pain on palpation absent
   2 = pain on palpation slight
   3 = pain on palpation moderate
   4 = pain on palpation severe
(ii) clinical examination to evaluate the degree of muscle tone (quadriceps muscles) with the use of the "Likert scale" as described by Innes (J.F. Innes et al., Journal of Small Animal Pratice, 39, 325-332, 1998). The scale of values used was from 0 to 3 and, in particular:
   0 = normal tone
   1 = slight atony
   2 = moderate atony
   3 = severe atony
(iii) examination with instruments, consisting of measuring the degree of osteophytosis by means of XR radiographic examination performed as described by Bouck (G.R. Bouck et al., Veterinary and Comparative Orthopaedic Traumatology, 8, 177-83, 1995). The scale of values used was from 1 to 4 and, in particular:
   1 = osteophytes absent
   2 = minimal and localized formation of osteophytes
   3 = moderate formation of osteophytes at several joint sites
   4 = serious osteophytosis affecting the entire joint
(iv) cytological examination of the synovial fluid, consisting of measuring a cell line characteristic of the inflammatory phenomenon; in particular, measurement of the number of leukocytes per litre of synovial fluid, performed as described by Gibson (N.R. Gibson et al., The Veterinary Record, April 24, 463-465, 1999), was selected. The values were expressed as the number of cells per litre of synovial fluid with a value of less than 3x10⁹ cells/litre for normal patients.

The evaluations were carried out on large dogs of various breeds and aged between 3 months and 7 years, which had had lameness in the right or left hind leg for no less than 2 months, due to DJD secondary to rupture of the cruciate ligament, dislocation of the kneecap (in puppies), or genu valgum (in puppies). The cases subjected to treatment are summarized in Table II.

**Table II**

| **Case** | **Breed** | **Sex** | **Age (years)** | **Weight (kg)** | **Primary pathological condition** |
|---|---|---|---|---|---|
| 1 | German Shepherd | male | 7 | 35 | ruptured |
| | | | | | cruciate |
| | | | | | ligament |
| 2 | Newfoundland | male | 1 | 30 | genu valgum |
| 3 | Chow Chow | female | 4 | 22 | ruptured |
| | | | | | cruciate |
| | | | | | ligament |
| 4 | German Shepherd | male | 5 | 36 | ruptured |
| | | | | | cruciate |
| | | | | | ligament |
| 5 | Alaskan Malamute | female | 3 months | 15 | dislocated |
| | | | | | kneecap |
| 6 | Boxer | female | 2 | 30 | Ruptured |
| | | | | | cruciate |
| | | | | | ligament |
| 7 | Chow Chow | male | 2 months | 15 | dislocated |
| | | | | | kneecap |
| 8 | Newfoundland | male | 4 | 48 | ruptured |
| | | | | | cruciate |
| | | | | | ligament |
| 9 | German Shepherd | female | 6 | 40 | ruptured |
| | | | | | cruciate |
| | | | | | ligament |
| 10 | Boxer | male | 3 | 34 | dislocated |
| | | | | | kneecap |
| 11 | German Shepherd | male | 2 | 25 | genu valgum |
| 12 | Maremma Sheepdog | female | 2 | 40 | ruptured |
| | | | | | cruciate |
| | | | | | ligament |

The treatments were carried out for a period of 40 days with daily administration of the drug *per os.*

The evaluations were made at time zero (to) and on the fortieth day (t₄₀).

The efficacy of the following compositions was evaluated by the preselected method:
Composition A: a combination of a specific molecular fraction of chondroitin sulphate characterized by an average molecular weight of between 5,000 and 10,000 Daltons (Compound B) with DL-α-lipoic acid. Tablets each contained:

| | |
|---|---|
| compound B | 150 mg |
| DL-α-lipoic acid | 20 mg |
| excipients to make up to | 700 mg |

Composition B: a combination of a specific molecular fraction of chondroitin sulphate characterized by an average molecular weight greater than 20,000 Daltons (compound E) with DL-α-lipoic acid. Tablets each contained:

| | |
|---|---|
| compound E | 150 mg |
| DL-α-lipoic acid | 20 mg |
| excipients to make up to | 700 mg |

Composition C: a composition based on phenylbutazone in tablet form. Tablets each contained:

| | |
|---|---|
| phenylbutazone | 50 mg |
| excipients to make up to | 700 mg |

Composition D: a composition based on a specific molecular fraction of chondroitin sulphate characterized by an average molecular weight of between 5,000 and 10,000 Daltons (compound B), but without thioctic acid. Each tablet contained:

| | |
|---|---|
| compound B | 150 mg |
| excipients to make up to | 700 mg. |

The results of the clinical tests are summarized in Table III.

**Table III**

| **case** | **composition** | **N0. administr./day** | **pain palpat.** | | **atony** | | **osteophytosis** | | **cytological examination** | |
|---|---|---|---|---|---|---|---|---|---|---|
| | | | **t0** | **t40** | **t0** | **t40** | **t0** | **t40** | **t0** | **t40** |
| 1 | A | 1 | 4 | 2 | 3 | 1 | 4 | 2 | 75 | 40 |
| 2 | A | 1 | 3 | 2 | 2 | 0 | 3 | 1 | 60 | 25 |
| 3 | A | 1 | 4 | 2 | 3 | 1 | 3 | 1 | 42 | 25 |
| 4 | B | 1 | 4 | 3 | 2 | 1 | 4 | 2 | 60 | 30 |
| 5 | B | 1 | 4 | 3 | 3 | 2 | 3 | 2 | 72 | 45 |
| 6 | B | 1 | 3 | 2 | 3 | 1 | 4 | 3 | 52 | 25 |
| 7 | C | 1 | 4 | 1 | 2 | 2 | 4 | 3 | 48 | 5 |
| 8 | C | 1 | 4 | 1 | 3 | 2 | 4 | 4 | 72 | 7 |
| 9 | C | 1 | 4 | 1 | 3 | 3 | 3 | 2 | 60 | 15 |
| 10 | D | 1 | 4 | 2 | 3 | 3 | 4 | 2 | 65 | 30 |
| 11 | D | 1 | 3 | 2 | 3 | 3 | 3 | 2 | 66 | 40 |
| 12 | D | 1 | 4 | 2 | 2 | 2 | 3 | 1 | 50 | 30 |

As can be seen from the data given in Table III, the compositions according to the present invention (Compositions A and B) have substantial advantages both in comparison with phenylbutazone (composition C), having truly anti-inflammatory activity, and in comparison with a composition containing chondroitin sulphate alone (composition D). The compositions of the invention, particularly composition A containing chondroitin sulphate with an average molecular weight of between 5,000 and 10,000 Daltons, were the only ones to show activity on muscle atony, thus constituting a real solution to the problem outlined above, that is, the need to act on the myo-tendinous-ligamentous morphofunctional unit (MTL-MFU) as a whole.

It can also be noted from the data in the table that composition A tends to have a more marked effect than composition B in the atony test. Bearing in mind that both compositions contain the same quantity of thioctic acid, the sole difference being the molecular fraction of chondroitin sulphate, and that the latter cannot affect muscle atony (see composition D), the result can only be explained by a synergistic effect between the two active components of the composition.

The use, in the compositions of the present invention, of molecular fractions of CS in which - by virtue of the purification method described below - the molecules having molecular weights greater than 30,000 Daltons have been eliminated and which, moreover, are free of fractions below 3,000 Daltons particularly enhances the chondroprotective effect.

On the basis of what has been demonstrated above, a further subject of the present invention is a molecular fraction of chondroitin sulphate which has an average molecular weight of 5,000-10,000 Daltons, which is free of chondroitin sulphate molecules with molecular weights less than or equal to 3,000 Daltons and, optionally, is free of chondroitin sulphate molecules with molecular weights greater than or equal to 30,000 Daltons, and its use for the preparation of a medicament or of a food supplement having chondroprotective activity for human or veterinary use.

A second further subject of the present invention is the use of an active ingredient selected from DL-alpha-lipoic acid or a pharmaceutically acceptable salt, ester or amide thereof, or an optical isomer thereof, for the prepartion of a medicament or of a food supplement for human or veterinary use, for the treatment of muscular atony associated with degenerative joint diseases.

The invention is described further by means of the following examples of pharmaceutical compositions. In the examples, compound A is the molecular fraction of chondroitin sulphate between 10,000 and 35,000 Daltons; compound B is the fraction of chondroitin sulphate between 5,000 and 10,000 Daltons, free of CS with molecular weight less than or equal to 3,000 Daltons and of CS with molecular weight greater than or equal to 30,000 Daltons; compound C is the fraction of chondroitin sulphate between 10,000 and 20,000 Daltons.

### Example 1 - ampoules for injection

| | |
|---|---|
| compound B | 100 mg |
| DL-alpha-lipoamide | 25 mg |
| sodium chloride | 17 mg |
| water for injections to make | up to 2 ml |

### Example 2 - lyophilized ampoules

| | |
|---|---|
| compound C | 50 mg |
| glucosamine hydrochloride | 100 mg |
| DL-alpha-lipoic acid, sodium salt | 30 mg |
| mannitol | 80 mg |
| Each solvent ampoule contains: | |
| water for injections to make | up to 2 ml |

### Example 3 - tablets

| | |
|---|---|
| compound A | 150 mg |
| DL-alpha-lipoic acid | 20 mg |
| lactose | 100 mg |
| maize starch | 70 mg |
| talc | 8 mg |
| magnesium stearate | 4 mg |
| carboxymethyl cellulose | 8 mg |

### Example 4 - tablets

| | |
|---|---|
| compound A | 250 mg |
| DL-alpha-lipoic acid | 35 mg |
| DL-alpha-tocopherol acetate | 50 mg |
| lactose | 150 mg |
| microcrystalline cellulose | 100 mg |
| talc | 12 mg |
| magnesium stearate | 7 mg |

### Example 5 - oily capsules

| | |
|---|---|
| compound B | 25 mg |
| compound C | 25 mg |
| DL-alpha-lipoic acid, micronized | 50 mg |
| glucosamine sulphate | 150 mg |
| maize oil | 150 mg |
| gelatine FU | 70 mg |
| glycerol FU | 30 mg |
| titanium dioxide (E171) | 0.5 mg |
| erythrosin (E127) | 1 mg |

Naturally, other pharmaceutical compositions containing a mixture of active ingredients according to the present invention together with pharmaceutically acceptable excipients may be provided. These compositions may be in the form of capsules, tablets, powders and pills, and in gastroresistant formulations for oral administration, and may also be produced with the preliminary use of microencapsulation, liposome incorporation and micellization techniques. For topical methods, including the transdermal method, formulations in suppositories, micro-enemas, creams, ointments, sprays, gels, sponges, dressings of various consistencies and patches may be used. All possible forms pharmaceutically indicated for the various methods of administration may also be formulated with excipients or technological processes suitable for producing fast-release or slow-release medicaments.

The dosages for the use of the compositions of the present invention may vary according to the method of administration and the severity of the disease and to the patient's general condition. The therapeutic dosages to be expected in man are in any case from 50 mg/day to 1,000 mg/day of chondroitin sulphate for a period of between 1 and 6 months of uninterrupted administration. In animals, particularly dogs, these therapeutic doses are from 10 mg/day to 500 mg/day of chondroitin sulphate. According to the severity of the condition, the treatment may be repeated in several cycles. Specific dosages or methods of treatment may be provided for by the physician according to the severity of the condition and the patient's physical condition.

A further subject of the present invention is a method of producing the molecular fractions of chondroitin sulphate indicated above as compounds, A, B, C, D and E with a high degree of purity, particularly a method of producing the molecular fraction of CS which has a molecular weight of between 5,000 and 10,000 Daltons and is free of chondroitin sulphate molecules with molecular weights less than or equal to 3,000 Daltons (compound B).

This method starts with commercial chondroitin sulphate, preferably of pig origin, having an average molecular weight of between 20,000 and 40,000 Daltons, a percentage of non-sulphurated disaccharide of between 2% and 10%, a percentage of disaccharide 4-sulphate of between 50% and 80%, a percentage of disaccharide 6-sulphate of between 15% and 40%, and a degree of sulphation per disaccharide of between 0.60 and 1.

This starting product is first of all dissolved in basic aqueous solution; the solution is heated in an autoclave to a pressure of between 1 and 1.2 atm and to a temperature of between 110°C and 150°C, preferably between 121°C and 135°C for 30 minutes, rapidly cooled to ambient temperature, neutralized, and filtered on celite. The clear solution thus obtained is filtered on a 1 µm membrane and poured into acetone. The precipitate is then separated by filtration and centrifugation and dried. The product obtained (compound A) has the following analytical characteristics: average molecular weight 10,000-35,000 Daltons degree of sulphation per disaccharide 0.75 - 1 protein content <1.5%

The compound A produced above is dissolved in water and the solution is subjected to constant-volume ultrafiltration on a 3,000 NMWL (nominal molecular weight limit) molecular exclusion membrane. Once a volume equal to 3.5 times the initial volume has been ultrafiltered, the filtrate is discarded (if desired, the molecular fraction contained may be recovered by being precipitated in acetone and dried; this fraction constitutes compound D); the solution remaining above the filter is subjected to further constant-volume ultrafiltration on a 10,000 NMWL molecular exclusion membrane. Once a volume equal to twice the starting volume has been ultrafiltered, the filtrate is concentrated and then precipitated in acetone and the precipitate is separated and dried. A product (compound B) having the following analytical characteristics is obtained:

| | |
|---|---|
| average molecular weight | 5,000-10,000 Daltons |
| degree of sulphation per disaccharide | 0.75-1 |
| protein content | <1.5% |

A fundamental characteristic of the compound B thus obtained is that it is free of CS molecules having molecular weights less than or equal to 3,000 Daltons. This result is achieved by means of the use of a 3,000 NMWL molecular exclusion membrane.

The solution which remains above the 10,000 NMWL membrane is subjected to ultrafiltration and concentration on a 30,000 NMWL molecular exclusion membrane. Once a volume equal to twice the initial volume has been ultrafiltered, (constant addition of 1 volume of water in the course of the ultrafiltration), the solution which has passed through the membrane is concentrated and then precipitated in acetone. The precipitate is separated and dried, producing a product (compound C) having the following analytical characteristics: average molecular weight 10,000-20,000 Daltons degree of sulphation per disaccharide 0.75 - 1. protein content <1.5%

The compound C thus obtained contains no chondroitin sulphate molecules having molecular weights greater than or equal to 30,000 Daltons, and this permits the production of an active ingredient in which the chondroprotective activity is maximized.

The product remaining above the filter is removed; if desired, the molecular fraction contained may be recovered by being precipitated in acetone and dried.

A possible variant of the method described above provides for the solution remaining above the 3,000 NMWL molecular exclusion membrane to be subjected directly to constant-volume ultrafiltration on a 30,000 Dalton molecular exclusion membrane. In this case, a molecular fraction (compound F) with an average molecular weight of between 5,000 and 20,000 Daltons, which is free of chondroitin sulphate molecules with molecular weights less than or equal to 3,000 Daltons and of chondroitin sulphate molecules with molecular weights greater than or equal to 30,000 Daltons will be obtained. This variant of the method may be economically advantageous, since it permits the recovery of a large fraction of product without this leading to drastic reductions in pharmacological activity.

The analytical characterization of the various molecular fractions of chondroitin sulphate was performed with the use of the following analytical methods.

Average molecular weight was determined by molecular exclusion chromatography (HPSEC) in accordance with the method described by N. Volpi and L Bolognini, J. Chromatogr., 630, 390-396 (1993).

Percentage of disaccharides was determined by strong anion exchange HPLC chromatography after treatment of the chondroitin sulphate molecular fractions with bacterial lyase in accordance with the method described by N. Volpi, I. Sandri and T. Venturelli, Carbohydr. Res. 279, 193-200 (1995).

Degree of sulphation was determined by ion-exchange chromatography (SAX-HPLC) in accordance with the method described by T. Imanari et al., J. Chromatogr., 720, 275-293 (1996).

Percentage of protein was determined by spectrophotometry in accordance with the method described by O.H. Lowry et al., J. Biol. Chem. 193, 265 (1951), modified by G.L. Peterson, Methods in Enzymology 91, 95 (1983).

The method outlined above is further described by the following examples of preparations, in which the separation of fraction E is also described.

### Example A - Preparation of compound A

100 g of commercial chondroitin sulphate (in the form of sodium salt) of pig origin, having the analytical characteristics set out above, was dissolved with stirring in 1 litre of 1N aqueous sodium hydroxide solution. The opaque solution thus obtained was heated in a steam autoclave to 121°C for 30 minutes at 1 atm and was then cooled to ambient temperature very rapidly and neutralized to pH 7.2 with 1N hydrochloric acid solution. The opaque solution was then filtered on a celite bed to give a clear, slightly straw-coloured liquid. Filtration was then carried out under pressure on a membrane with a degree of filtration of 1 µm (a Millipore POLYGARD-CT 01 polypropylene cartridge and a differential pressure of between 3 and 3.5 bars were used). The completely clear solution thus obtained was poured into 5 litres of anhydrous acetone at ambient temperature with stirring. Slow stirring was continued for 2 hours, producing a flaky white precipitate which tended to settle. The precipitate was recovered by filtration and then dried under vacuum. 95 g of compound A, conforming to the analytical characteristics given above, was obtained.

### Example B - Preparation of compound B

A 10% aqueous solution of compound A, prepared as indicated in Example A, was pre-filtered through a polypropylene cartridge with a degree of filtration of 100 µm (a Millipore POLYGARD-CT 99 cartridge) and then subjected to constant-volume ultrafiltration on 3,000 NMWL molecular exclusion regenerated cellulose membrane (Millipore PLBC membranes premounted on Millipore PELLICON modules with tangential flow were used). The process was carried out at a temperature of between 20 and 25°C with the use of a differential pressure of 6 bars, achieved by means of a peristaltic pump (a Millipore EASY-LOAD pump). The pump was mounted so as to be able to draw the solution to be ultrafiltered from the container, in which stirring was maintained and into which water was admitted continuously in a volume equal to that of the ultrafiltrate. Once a volume equal to 3.5 times the initial volume had been ultrafiltered, the filtrate was discarded (or treated as described in Example D) and the solution remaining above the membrane was subjected to constant-volume ultrafiltration on a 10,000 NMWL molecular exclusion composite regenerated cellulose membrane (Millipore PLCGC membranes premounted on PELLICON modules with tangential flow were used). In this case also, the ultrafiltration was carried out at a temperature of between 20 and 25°C with the use of a differential pressure of 6 bars, achieved by means of the apparatus described above. Once a volume equal to twice the initial volume had been ultrafiltered, the solution which had passed through the membrane was concentrated to half its volume and precipitated by being poured into 5 volumes of anhydrous acetone at ambient temperature, with stirring. Slow stirring was continued for 2 hours thus producing a flaky white precipitate which tended to settle. The precipitate was recovered by filtration and was then dried under vacuum. 60 g of compound B having the analytical characteristics given above was obtained.

### Example C - Preparation of compound C

The solution which remained above the 10,000 NMWL membrane during the preparation of Example B was subjected to further constant-volume ultrafiltration on a 30,000 NMWL molecular exclusion composite regenerated cellulose membrane (a Millipore PLCTK membrane premounted on PELLICON modules with tangential flow was used). In this case also, the process was carried out at a temperature of between 20 and 25°C with the use of a differential pressure of 6 bars, achieved by means of the apparatus described above. Once a volume equal to twice the initial volume had been ultrafiltered, the ultrafiltered solution was concentrated to half its volume and precipitated by being poured into 5 volumes of anhydrous acetone at ambient temperature, with stirring. Slow stirring was continued for 2 hours thus producing a flaky white precipitate which tended to settle. The precipitate was recovered by filtration and was then dried under vacuum, producing 20 g of compound C having the analytical characteristics given above.

### Example D - preparation of compound D

The solution ultrafiltered through the 3,000 NMWL membrane (preparation of example B) was concentrated to half its volume, in small quantities, and precipitated in 5 volumes of anhydrous acetone. Slow stirring was continued for 2 hours thus producing a flaky white precipitate which tended to settle. The precipitate was recovered by filtration and was then dried under vacuum.

### Example E - preparation of compound E

The solution remaining above the 30,000 NMWL membrane in the preparation of Example C was concentrated to half its volume and precipitated in 5 volumes of anhydrous acetone. Slow stirring was continued for 2 hours thus producing a flaky white precipitate which tended to settle. The precipitate was recovered by filtration and was then dried under vacuum. 10 g of compound E of molecular weight greater than 20,000 Daltons was obtained.

## Claims

1. A pharmaceutical composition comprising a mixture of chondroitin sulphates and of an active ingredient selected from DL-alpha-lipoic acid, a pharmaceutically acceptable salt, ester or amide thereof, or an optical isomer thereof, **characterized in that** the said mixture of chondroitin sulhates is a molecular fraction which has an average molecular weight of between 5,000 and 10,000 Daltons and which is free of chondroitin sulphates of molecular weight less than or equal to 3,000 Daltons.

2. A composition according to Claim 1, in which the chondroitin sulphate and the active ingredient are in a proportion by weight of chondroitin sulphate/active ingredient variable from 50:1 to 1:2.

3. A composition according to Claim 2, in which the proportion by weight between chondroitin sulphate and the active ingredient is between 10:1 and 1:1.

4. A composition according to any one of Claims 1 to 3, in which the chondroitin sulphate is a mixture of chondroitin 4-sulphate, in a proportion variable between 50% and 80% by weight, and chondroitin 6-sulphate, in a proportion variable between 15% and 40% by weight, having a degree of sulphation per disaccharide of between 0.60 and 1 and a percentage of non-sulphurated disaccharide of between 2% and 10%.

5. A composition according to any one of Claims 1 to 4, in which the chondroitin sulphate has a degree of sulphation per disaccharide of between 0.75 and 1 and a protein content of less than 1.5% by weight.

6. A composition according to Claim 5, in which the protein content is between 0.05% and 1.2% by weight.

7. A composition according to any one of Claims 1 to 6, in which the active ingredient is DL-alpha-lipoic acid or a salt thereof with an alkali-metal or an alkaline-earth metal.

8. A composition according to Claim 7, in which the salt of DL-alpha-lipoic acid is a sodium salt.

9. A composition according to any one of Claims 1 to 8, comprising, in addition, one or more substances selected from:
- an amino sugar,
- a manganese salt,
- an antioxidant.

10. A composition according to Claim 9, in which the amino sugar is glucosamine in a ratio by weight of between 1:1 and 4:1, relative to the chondroitin sulphate.

11. A composition according to Claim 9 or Claim 10, in which the manganese salt is manganese ascorbate in a ratio by weight of between 1:3 and 1:10, relative to the chondroitin sulphate.

12. A composition according to any one of Claims 9 to 11, in which the antioxidant is vitamin E in a ratio by weight of between 1:7 and 1:3, relative to the chondroitin sulphate.

13. A composition according to any one of Claims 9 to 11, in which the antioxidant is vitamin C in a ratio by weight of between 1:10 and 1:1, relative to the chondroitin sulphate.

14. A composition according to any one of Claims 9 to 11, in which the antioxidant is a flavonoid, preferably quercetin or rutin, in a ratio by weight of between 1:5 and 1:1, relative to the chondroitin sulphate.

15. Use of a composition according to any one of Claims 1 to 14 for the preparation of a medicament or a food supplement for human or veterinary use for the treatment of degenerative joint diseases.

16. A molecular fraction of chondroitin sulphate having an average molecular weight of between 5,000 and 10,000 Daltons, the molecular fraction being **characterized in that** it is free of chondroitin sulphate molecules with molecular weight less than or equal to 3,000 Daltons.

17. Use of the molecular fraction according to Claim 16 for the preparation of a medicament or a food supplement with chondroprotective activity, for human or veterinary use.

18. A method of preparing the fraction of chondroitin sulphate according to Claim 16 from commercial chondroitin sulphate, the method comprising the steps of:
(a) dissolving the commercial chondroitin sulphate in basic aqueous solution and heating the solution at a pressure of between 1 and 1.2 atm and to a temperature of between 110°C and 150°C;
(b) cooling the solution obtained in step (a) to ambient temperature, neutralizing it, filtering it to clearness and precipitating the clear solution in acetone, obtaining compound A having an average molecular weight of 10,000-35,000 Daltons, a degree of sulphation per disaccharide of between 0.75 - 1 and a protein content of <1.5%;
(c) dissolving the compound A obtained in step (b) in water and subjecting the solution to constant-volume ultrafiltration on 3,000 NMWL molecular exclusion membrane, ultrafiltering a volume equal to 3.5 times the initial volume,
(d) subjecting the solution remaining above the filter in step (c) to constant-volume ultrafiltration on a 10,000 NMWL molecular exclusion membrane, ultrafiltering a volume equal to twice the initial volume,
(e) precipitating the filtrate obtained in step (d) with acetone, optionally after concentration, obtaining compound B having an average molecular weight of 5,000-10,000 Daltons, a degree of sulphation per disaccharide of 0.75 - 1, and a protein content of <1.5%, the compound B being free of chondroitin sulphate molecules with molecular weights less than or equal to 3,000 Daltons.

19. A method according to Claim 18, in which the commercial chondroitin sulphate is of pig origin and has an average molecular weight of between 20,000 and 40,000 Daltons.

20. A method according to Claim 18 or Claim 19, in which the commercial chondroitin sulphate solution is heated, in step (a), to between 121°C and 135°C for 30 minutes.

21. A method according to any one of Claims 18 to 20, in which the filtration of the solution obtained in step (a) is performed on celite and/or on a 1 µm membrane.

22. A method according to any one of Claims 18 to 21, the method further comprising the steps of:
(f) subjecting the solution remaining above the 10,000 NMWL membrane in step (d) to ultrafiltration to concentration on 30,000 NMWL molecular exclusion membrane, ulterfiltering a volume equal to twice the initial volume,
(g) precipitating the filtrate obtained in step (f) in acetone, optionally after concentration, obtaining compound C having an average molecular weight of 10,000-20,000 Daltons, a degree of sulphation per disaccharide of 0.75 - 1, and a protein content of <1.5%.

23. A method according to any one of Claims 18 to 21, in which the constant-volume ultrafiltration in step (d) is performed on 30,000 NMWL molecular exclusion membrane producing, after step (e), compound F having an average molecular weight of 5,000-20,000 Daltons, a degree of sulphation per disaccharide of 0.75 - 1, and a protein content of <1.5%, the compound F being free of chondroitin sulphate molecules with molecular weight less than or equal to 3,000 Daltons and of chondroitin sulphate molecules with molecular weight greater than or equal to 30,000 Daltons.

## Patentansprüche

1. Pharmazeutische Zusammensetzung, umfassend ein Gemisch aus Chondroitinsulfaten und einem wirksamen Bestandteil, ausgewählt aus DL-α-Liponsäure, einem pharmazeutisch geeigneten Salz, Ester oder Amid davon, oder einem optischen Isomer davon, **dadurch gekennzeichnet, dass** das Gemisch aus Chondroitinsulfaten eine Molekülfraktion ist, die eine mittlere Molmasse zwischen 5000 und 10000 Dalton hat und frei ist von Chondroitinsulfaten mit einer Molmasse kleiner oder gleich 3000 Dalton.

2. Zusammensetzung nach Anspruch 1, worin das Chondroitinsulfat und der wirksame Bestandteil in einem Chondroitinsulfat/wirksamer Bestandteil-Gewichtsverhältnis vorliegen, das von 50:1 bis 1:2 variabel ist.

3. Zusammensetzung nach Anspruch 2, worin das Gewichtsverhältnis zwischen Chondroitinsulfat und dem wirksamen Bestandteil zwischen 10:1 und 1:1 liegt.

4. Zusammensetzung nach einem der Ansprüche 1 bis 3, worin das Chondroitinsulfat ein Gemisch ist aus Chondroitin-4-sulfat in einem Verhältnis, das zwischen 50 und 80 Gew.-% variiert, und Chondroitin-6-sulfat in einem Verhältnis, das zwischen 15 und 40 Gew.-% variiert, und das einen Sulfatierungsgrad pro Disaccharid von 0,60 bis 1 und einen Prozentgehalt an nichtgeschwefeltem Disaccharid zwischen 2% und 10% hat.

5. Zusammensetzung nach einem der Ansprüche 1 bis 4, worin das Chondroitinsulfat einen Sulfatierungsgrad pro Disaccharid zwischen 0,75 und 1 und einen Proteingehalt von weniger als 1,5 Gew.-% hat.

6. Zusammensetzung nach Anspruch 5, worin der Proteingehalt zwischen 0,05 und 1,2 Gew.-% liegt.

7. Zusammensetzung nach einem der Ansprüche 1 bis 6, worin der wirksame Bestandteil DL-α-Liponsäure oder deren Salz mit einem Alkalimetall oder einem Erdalkalimetall ist.

8. Zusammensetzung nach Anspruch 7, worin das Salz der DL-α-Liponsäure ein Natriumsalz ist.

9. Zusammensetzung nach einem der Ansprüche 1 bis 8, umfassend zusätzlich eine oder mehrere Substanzen, die ausgewählt sind aus:
- einem Aminozucker
- einem Mangansalz
- einem Antioxidationsmittel.

10. Zusammensetzung nach Anspruch 9, worin der Aminozucker Glucosamin in einem Gewichtsverhältnis zwischen 1:1 und 4:1, bezogen auf das Chondroitinsulfat, ist.

11. Zusammensetzung nach Anspruch 9 oder Anspruch 10, worin das Mangansalz Manganascorbat in einem Gewichtsverhältnis zwischen 1:3 und 1:10, bezogen auf das Chondroitinsulfat, ist.

12. Zusammensetzung nach einem der Ansprüche 9 bis 11, worin das Antioxidationsmittel Vitamin E in einem Gewichtsverhältnis zwischen 1:7 und 1:3, bezogen auf das Chondroitinsulfat, ist.

13. Zusammensetzung nach einem der Ansprüche 9 bis 11, worin das Antioxidationsmittel Vitamin C in einem Gewichtsverhältnis zwischen 1:10 und 1:1, bezogen auf das Chondroitinsulfat, ist.

14. Zusammensetzung nach einem der Ansprüche 9 bis 11, worin das Antioxidationsmittel ein Flavonoid, vorzugsweise Quercetin oder Rutin, in einem Gewichtsverhältnis zwischen 1:5 und 1:1, bezogen auf das Chondroitinsulfat, ist.

15. Verwendung einer Zusammensetzung gemäß einem der Ansprüche 1 bis 14 zur Herstellung eines Medikaments oder Nahrungsergänzungsmittels zur ärztlichen oder tierärztlichen Verwendung bei der Behandlung von degenerativen Gelenkerkrankungen.

16. Molekülfraktion von Chondroitinsulfat mit einer mittleren Molmasse zwischen 5000 und 10000 Dalton, worin die Molekülfraktion **dadurch gekennzeichnet ist, dass** sie frei ist von Chondroitinsulfatmolekülen mit Molmassen kleiner oder gleich 3000 Dalton.

17. Verwendung der Molekülfraktion nach Anspruch 16 zur Herstellung eines Medikaments oder Nahrungsergänzungsmittels mit knorpelschützender Wirkung zur ärztlichen oder tierärztlichen Verwendung.

18. Verfahren zur Herstellung der Chondroitinsulfatfraktion nach Anspruch 16 aus kommerziellem Chondroitinsulfat, wobei das Verfahren die folgenden Schritte umfasst:
(a) Auflösen des kommerziellen Chondroitinsulfats in einer basischen, wässrigen Lösung und Erhitzen der Lösung bei einem Druck von 1 bis 1,2 atm auf eine Temperatur von 110 bis 150°C;
(b) Abkühlen der in Schritt (a) erhaltenen Lösung auf Raumtemperatur, Neutralisieren, Filtrieren bis zur Klärung und Ausfällen der klaren Lösung in Aceton, wodurch eine Verbindung A mit einer mittleren Molmasse von 10000-35000 Dalton, einem Sulfatierungsgrad pro Disaccharid von 0,75-1 und einem Proteingehalt von <1,5% erhalten wird;
(c) Auflösen der in Schritt (b) erhaltenen Verbindung A in Wasser und Ultrafiltration der Lösung bei konstantem Volumen über eine 3000 NMWL-Molekularsiebmembran, wobei ein Volumen gleich dem 3,5-fachen des Anfangsvolumens ultrafiltriert wird,
(d) Ultrafiltration der in Schritt (c) über dem Filter verbleibenden Lösung bei konstantem Volumen über eine 10000 NMWL-Molekularsiebmembran, wobei ein Volumen gleich dem Doppelten des Anfangsvolumens ultrafiltriert wird,
(e) Ausfällen des in Schritt (d) erhaltenen Filtrats mit Aceton, wahlweise nach Konzentrieren, wodurch eine Verbindung B mit einer mittleren Molmasse von 5000-10000 Dalton, einem Sulfatierungsgrad pro Disaccharid von 0,75-1 und einem Proteingehalt von <1,5% erhalten wird, wobei die Verbindung B frei ist von Chondroitinsulfatmolekülen mit Molmassen kleiner oder gleich 3000 Dalton.

19. Verfahren nach Anspruch 18, bei dem das kommerzielle Chondroitinsulfat aus Schweinen stammt und eine mittlere Molmasse von 20000 bis 40000 Dalton hat.

20. Verfahren nach Anspruch 18 oder Anspruch 19, bei dem die Lösung des kommerziellen Chondroitinsulfats in Schritt (a) 30 Minuten lang auf 121 bis 135°C erhitzt wird.

21. Verfahren nach einem der Ansprüche 18 bis 20, bei dem die Flitration der in Schritt (a) erhaltenen Lösung über Celite und/oder eine 1 µm-Membran durchgeführt wird.

22. Verfahren nach einem der Ansprüche 18 bis 21, wobei das Verfahren weiterhin die folgenden Schritte umfasst:
(f) Ultrafiltration der über der 10000 NMWL-Membran verbleibenden Lösung in Schritt (d) bei konstantem Volumen über eine 30000 NMWL-Molekularsiebmembran, wobei ein Volumen gleich dem Doppelten des Anfangsvolumens ultrafiltriert wird,
(g) Ausfällen des in Schritt (f) erhaltenen Filtrats mit Aceton, wahlweise nach Konzentrieren, wodurch eine Verbindung C mit einer mittleren Molmasse von 10000-20000 Dalton, einem Sulfatierungsgrad pro Disaccharid von 0,75-1 und einem Proteingehalt von <1,5% erhalten wird.

23. Verfahren nach einem der Ansprüche 18 bis 21, bei dem die Ultrafiltration bei konstantem Volumen in Schritt (d) über eine 30000 NMWL-Molekularsiebmembran durchgeführt wird, wodurch nach dem Schritt (e) eine Verbindung F mit einer mittleren Molmasse von 5000-20000 Dalton, einem Sulfatierungsgrad pro Disaccharid von 0,75-1 und einem Proteingehalt von <1,5% erhalten wird, wobei die Verbindung F frei ist von Chondroitinsulfatmolekülen mit Molmassen kleiner oder gleich 3000 Dalton sowie von Chondroitinsulfatmolekülen mit Molmassen grösser oder gleich 30000 Dalton.

## Revendications

1. Composition pharmaceutique comprenant un mélange de sulfates de chondroitine et d'un ingrédient actif choisi à partir de l'acide DL-alpha-lipoïque, un de ses sels, ester ou amide pharmaceutiquement acceptables, ou un de ses isomères optiques, **caractérisée en ce que** ledit mélange de sulfates de chondroitine est une fraction moléculaire qui a un poids moléculaire moyen d'entre 5 000 et 10 000 daltons et qui est exempt de sulfates de chondroitine de poids moléculaire inférieur ou égal à 3 000 daltons.

2. Composition selon la revendication 1, dans laquelle le sulfate de chondroitine et l'ingrédient actif sont dans une proportion en poids de sulfate de chondroïtine/ingrédient actif variable de 50:1 à 1:2.

3. Composition selon la revendication 2, dans laquelle la proportion en poids entre le sulfate de chondroitine et l'ingrédient actif est entre 10:1 et 1:1.

4. Composition selon l'une quelconque des revendications 1 à 3, dans laquelle le sulfate de chondroïtine est un mélange de 4-sulfate de chondroïtine, dans une proportion variable entre 50 % et 80 % en poids, et de 6-sulfate de chondroïtine, dans une proportion variable entre 15 % et 40 % en poids, ayant un degré de sulfatation par disaccharide entre 0,60 et 1 et un pourcentage de disaccharide non sulfuré entre 2 % et 10 %.

5. Composition selon l'une quelconque des revendications 1 à 4, dans laquelle le sulfate de chondroïtine a un degré de sulfatation par disaccharide entre 0,75 et 1 et une teneur en protéines inférieure à 1,5 % en poids.

6. Composition selon la revendication 5, dans laquelle la teneur en protéines est entre 0,05 % et 1,2 % en poids.

7. Composition selon l'une quelconque des revendications 1 à 6, dans laquelle l'ingrédient actif est l'acide DL-alpha-lipoïque ou un de ses sels avec un métal alcalin ou un métal alcalino-terreux.

8. Composition selon la revendication 7, dans laquelle le sel de l'acide DL-alpha-lipoïque est un sel de sodium.

9. Composition selon l'une quelconque des revendications 1 à 8, comprenant, en plus, une ou plusieurs substances sélectionnées à partir de :
- une osamine,
- un sel de manganèse,
- un antioxydant.

10. Composition selon la revendication 9, dans laquelle l'osamine est la glucosamine dans un rapport en poids entre 1:1 et 4:1, par rapport au sulfate de chondroïtine.

11. Composition selon la revendication 9 ou la revendication 10, dans laquelle le sel de manganèse est de l'ascorbate de manganèse dans un rapport en poids d'entre 1:3 et 1:10, par rapport au sulfate de chondroïtine.

12. Composition selon l'une quelconque des revendications 9 à 11, dans laquelle l'antioxydant est la vitamine E dans un rapport en poids entre 1:7 et 1:3, par rapport au sulfate de chondroïtine.

13. Composition selon l'une quelconque des revendications 9 à 11, dans laquelle l'antioxydant est la vitamine C dans un rapport en poids entre 1:10 et 1:1, par rapport au sulfate de chondroitine.

14. Composition selon l'une quelconque des revendications 9 à 11, dans laquelle l'antioxydant est un flavonoïde, de préférence la quercétine ou la rutine, dans un rapport en poids entre 1:5 et 1:1, par rapport au sulfate de chondroïtine.

15. Utilisation d'une composition selon l'une quelconque des revendications 1 à 14 pour la préparation d'un médicament ou d'un complément alimentaire pour l'utilisation humaine ou vétérinaire pour le traitement d'affections dégénératives des articulations.

16. Fraction moléculaire de sulfate de chondroïtine ayant un poids moléculaire moyen entre 5 000 et 10 000 daltons, la fraction moléculaire étant **caractérisée en ce qu'**elle est exempte de molécules de sulfate de chondroïtine avec poids moléculaire inférieur ou égal à 3 000 daltons.

17. Utilisation de la fraction moléculaire selon la revendication 16 pour la préparation d'un médicament ou d'un complément alimentaire avec une activité chondroprotectrice, pour l'utilisation humaine ou vétérinaire.

18. Procédé de préparation de la fraction de sulfate de chondroitine selon la revendication 16 à partir de sulfate de chondroïtine du commerce, le procédé comprenant les étapes consistant à :
(a) dissoudre le sulfate de chondroïtine du commerce dans une solution aqueuse basique et chauffer la solution à une pression entre 1 et 1,2 atm et à une température entre 110° et 150 °C ;
(b) refroidir la solution obtenue dans l'étape (a) à température ambiante, la neutraliser, la filtrer jusqu'à ce qu'elle soit claire et précipiter la solution claire dans de l'acétone, pour obtenir un composé A ayant un poids moléculaire moyen de 10 000 à 35 000 daltons, à un degré de sulfatation par disaccharide entre 0,75 et 1 et une teneur en protéines de < 1,5 % ;
(c) dissoudre le composé A obtenu dans l'étape (b) dans de l'eau et soumettre la solution à une ultrafiltration à volume constant sur une membrane d'exclusion moléculaire de NMWL 3 000, en ultrafiltrant un volume égal à 3,5 fois le volume initial,
(d) soumettre la solution restante au-dessus du filtre dans l'étape (c) à une ultrafiltration à volume constant sur une membrane d'exclusion moléculaire de NMWL 10 000, en ultrafiltrant un volume égal à deux fois le volume initial,
(e) précipiter le filtrat obtenu dans l'étape (d) avec de l'acétone, éventuellement après concentration, pour obtenir un composé B ayant un poids moléculaire moyen de 5 000 à 10 000 daltons, un degré de sulfatation par disaccharide de 0,75 à 1, et une teneur en protéines de < 1,5 %, le composé B étant exempt de molécules de sulfate de chondroïtine avec des poids moléculaires inférieurs ou égaux à 3 000 daltons.

19. Procédé selon la revendication 18, dans lequel le sulfate de chondroïtine du commerce est d'origine porcine et a un poids moléculaire moyen entre 20 000 et 40 000 daltons.

20. Procédé selon la revendication 18 ou la revendication 19, dans lequel la solution de sulfate de chondroitine est chauffée, dans l'étape (a) à une température entre 121 °C et 135 °C pendant 30 minutes.

21. Procédé selon l'une quelconque des revendications 18 à 20, dans lequel la filtration de la solution obtenue dans l'étape (a) est effectuée sur célite et/ou sur une membrane de 1 µm.

22. Procédé selon l'une quelconque des revendications 18 à 21, le procédé comprenant en outre les étapes consistant à :
(f) soumettre la solution restant sur la membrane de NMWL 10 000 dans l'étape (d) à une ultrafiltration jusqu'à une certaine concentration sur une membrane d'exclusion moléculaire de NMWL 30 000, en ultrafiltrant un volume égal à deux fois le volume initial,
(g) précipiter le filtrat obtenu dans l'étape (f) dans de l'acétone, éventuellement après concentration, pour obtenir un composé C ayant un poids moléculaire moyen de 10 000 à 20 000 daltons, un degré de sulfatation par disaccharide de 0,75 à 1, et une teneur en protéines de < 1,5 %.

23. Procédé selon l'une quelconque des revendications 18 à 21, dans lequel l'ultrafiltration à volume constant dans l'étape (d) est effectuée sur une membrane d'exclusion moléculaire de NMWL 30 000 produisant, après l'étape (e), un composé F ayant un poids moléculaire moyen de 5 000 à 20 000 daltons, un degré de sulfatation par disaccharide de 0,75 à 1, et une teneur en protéines de < 1,5 %, le composé F étant exempt de molécules de sulfate de chondroitine avec un poids moléculaire inférieur ou égal à 3 000 daltons et de molécules de sulfate de chondroïtine avec un poids moléculaire supérieur ou égal à 30 000 daltons.
